# EUROPÄISCHE PATENTSCHRIFT

(11) **EP 2 810 610 B1**
(45) Veröffentlichungstag und Bekanntmachung des Hinweises auf die Patenterteilung: **08.08.2018**
(21) Anmeldenummer: 13170974.3
(22) Anmeldetag: 07.06.2013
(51) Int. Cl.: A61B 17/80

(54) **Knochenplatte**
Bone plate
Plaque de cuisson

(43) Veröffentlichungstag der Anmeldung: 10.12.2014
(73) Patentinhaber: Wolter, Dietmar, 22955 Hoisdorf (DE)
(72) Erfinder: Wolter, Dietmar, 22955 Hoisdorf (DE)
(74) Vertreter: Glawe, Delfs, Moll

(56) Entgegenhaltungen:
- EP-A2- 1 741 397
- EP-B1- 1 211 994
- US-A- 4 219 015

## Beschreibung

Die Erfindung betrifft eine Knochenplatte mit einer Mehrzahl von Durchgangslöchern. Die Durchgangslöcher erstrecken sich von einer Oberseite bis zur einer knochenseitigen Unterseite der Knochenplatte. Die Knochenplatte umfasst einen Plattensteg, der sich über eine Bruchstelle des Knochens hinweg erstreckt, wenn die Knochenplatte bestimmungsgemäß mit dem Knochen verbunden ist. Die Knochenplatte umfasst ein zweites proximales Durchgangsloch, das einen größeren Abstand zu dem Plattensteg hat als das erste proximale Durchgangsloch. Die Knochenplatte umfasst außerdem ein drittes proximales Durchgangsloch, das einen größeren Abstand zu dem Plattensteg hat als das zweite proximale Durchgangsloch. Das erste proximale Durchgangsloch weist eine Sitzfläche auf, die für einen winkelstabilen Eingriff einer Knochenschraube ausgelegt ist.

Solche Knochenplatten dienen dazu, einen Knochen nach einem Bruch zu stabilisieren. Dazu wird die Knochenplatte so positioniert, dass der Plattensteg sich über die Bruchstelle hinweg erstreckt. Der Plattensteg kann unterschiedliche Längen aufweisen. In der kürzesten Version ist der Plattensteg so lang wie der herkömmliche Lochabstand. Durch die proximalen Durchgangslöcher wird die Knochenplatte mit einem proximalen Knochenfragmente verbunden. Die distalen Durchgangslöcher dienen der Verbindung mit einem distalen Knochenfragment. Die Bruchstelle wird dadurch ruhig gestellt und der Knochen kann ausheilen. Zum Verbinden der Knochenplatte mit dem Knochen werden Knochenschrauben verwendet, die durch die Durchgangslöcher hindurchgeführt werden und in das Knochenmaterial eingeschraubt werden.

Die nahe der Bruchstelle angeordneten Knochenschrauben können winkelstabil in der Knochenplatte verriegelt sein. Dadurch werden die Lasten vermindert, die zwischen dem Schaft der Knochenschraube und dem Knochematerial übertragen werden. Allerdings hat die winkelstabile Verriegelung zur Folge, dass über das betreffende Durchgangsloch sehr hohe Lasten übertragen werden.
Versuche haben gezeigt, dass durch die winkelstabile Verriegelung die über das erste proximale Durchgangsloch übertragenen Lasten etwa doppelt so groß sind wie die über das zweite proximale Durchgangsloch übertragenen Lasten, die ihrerseits wieder etwa doppelt so groß sind wie die über das dritte proximale Durchgangsloch übertragenen Lasten. Dabei beziehen sich die Lastangaben auf die Kräfte, die über eine in das betreffende Durchgangsloch eingesetzte Knochenschraube auf den Knochen übertragen werden.

Der unterschiedlichen Lastenverteilung wurde im Stand der Technik beispielsweise dadurch Rechnung getragen, dass die Knochenplatten im Bereich der Durchgangslöcher, die besonders hohe Lasten aufnehmen, mit einer erhöhten Stabilität gestaltet wurden, EP 1 211 994 A2.

Der Erfindung liegt die Aufgabe zu Grunde, eine Knochenplatte vorzustellen, die eine gleichmäßigere Übertragung von Lasten auf den Knochen ermöglicht. Die Aufgabe wird gelöst mit den Merkmalen des Anspruchs 1. Vorteilhafte Ausführungsformen finden sich in den Unteransprüchen.

Erfindungsgemäß zeichnet sich die Knochenplatte dadurch aus, dass in einer Projektion auf eine Längsmittellinie der Knochenplatte der Abstand zwischen dem ersten proximalen Durchgangsloch und dem zweiten proximalen Durchgangsloch (erster proximaler Abstand) größer ist als der Abstand zwischen dem zweiten proximalen Durchgangsloch und dem dritten proximalen Durchgangsloch (zweiter proximaler Abstand).

Zunächst werden einige Begriffe erläutert. Die Längsmittellinie der Knochenplatte erstreckt sich von dem proximalen Ende der Knochenplatte bis zum distalen Ende der Knochenplatte. Die Längsmittellinie ist mittig in der Knochenplatte angeordnet, so dass der Abstand von der Längsmittellinie zum seitlichen Rand der Knochenplatte in beiden Richtungen gleich ist.

Die Projektion eines Durchgangslochs auf die Längsmittellinie bezeichnet eine gedachte Position des Durchgangslochs, bei der das Durchgangsloch so verschoben ist, dass der Mittelpunkt des Durchgangslochs auf der Längsmittellinie liegt. Die Verschiebung erfolgt entlang einer Geraden, die mit der Längsmittellinie einen rechten Winkel einschließt und sich durch den Mittelpunkt des Durchgangslochs in der Ausgangsposition erstreckt.

Der Abstand zweier Durchgangslöcher bezeichnet die entlang der Längsmittellinie gemessene Strecke, durch die die Projektionen der Durchgangslöcher voneinander getrennt sind. Dies entspricht der Länge der Längsmittellinie zwischen den beiden einander zugewandten Enden der Projektionen der beiden Durchgangslöcher.

Die Durchgangslöcher, auf die sich die Abstandsangaben beziehen, sind jeweils benachbart zueinander. Die Längsmittellinie schneidet also zwischen zwei benachbarten Durchgangslöchern kein weiteres Durchgangsloch bzw. keine Projektion eines weiteren Durchgangslochs, wenn alle Durchgangslöcher der Knochenplatte auf die Längsmittellinie projiziert sind.

Die proximalen Durchgangslöcher sind durch den Plattensteg von den distalen Durchgangslöchern getrennt. Wenn die Knochenplatte mit dem Knochen verbunden ist, sind also die proximalen Durchgangslöcher mit einem anderen Knochenfragment verbunden als die distalen Durchgangslöcher. Regelmäßig wird über die proximalen Durchgangslöcher eine Verbindung zu einem proximalen Fragment des Knochens und über die distalen Durchgangslöcher eine Verbindung zu einem distalen Fragment des Knochens hergestellt. Im Zusammenhang des Knochens beziehen sich die Begriffe proximal und distal auf den Abstand vom Körpermittelpunkt.

Bei einem winkelstabilen Eingriff ist die Achse der Knochenschraube relativ zu der Achse des Durchgangslochs alleine durch den Eingriff zwischen der Knochenschraube und der Sitzfläche des Durchgangslochs eindeutig definiert. Dies kann beispielsweise durch einen Gewindeingriff zwischen dem Kopf der Knochenschraube und der Sitzfläche des Durchgangslochs erreicht werden.

Die Erfindung hat erkannt, dass die Last, die über das zu der Bruchstelle benachbarte Durchgangsloch (erstes proximales Durchgangsloch) übertragen wird, sich vermindert, wenn der Abstand zum nächsten Durchgangsloch (zweites proximales Durchgangsloch) vergrößert wird. Das erste proximale Durchgangsloch hat also eine vergleichsweise isolierte Position zwischen dem Plattensteg und dem zweiten proximalen Durchgangsloch. Die erfindungsgemäße Lehre setzt sich damit in Gegensatz zu der gängigen Vorgehensweise, den Bereich, in dem die höchsten Belastungen auftreten, mit möglichst vielen Befestigungselementen zu versehen. Stattdessen ist bei der Erfindung im Bereich der höchsten Belastung lediglich ein einzelnes Durchgangsloch mit einer einzelnen winkelstabil verriegelten Knochenschraube vorgesehen, während ein sich daran anschließender Bereich gezielt von weiteren Befestigungsmitteln freigehalten wird. Möglicherweise wirkt sich der verlängerte Hebelarm zwischen dem ersten proximalen Durchgangsloch und dem zweiten proximalen Durchgangsloch positiv aus, indem dem Knochen und der Knochenplatte eine gewisse gemeinsame Bewegungsfreiheit ermöglicht wird.

Der Faktor, um den der erste proximale Abstand größer ist als der zweite proximale Abstand, kann zwischen 1,2 und 2, vorzugsweise zwischen 1,4 und 1,8 liegen. Der erste proximale Abstand kann beispielsweise zwischen 15 mm und 30 mm betragen.

Vorzugsweise hat die Knochenplatte auch auf der distalen Seite des Plattenstegs eine Mehrzahl von Durchgangslöchern. In einer vorteilhaften Ausführungsform ist der Abstand zwischen dem ersten distalen Durchgangsloch und dem zweiten distalen Durchgangsloch (erster distale Abstand) genauso groß wie der erste proximale Abstand. Durch eine solche Symmetrie in der Knochenplatte wird die gleichmäßige Kraftübertragung auf den Knochen weiter gefördert. Das erste distale Durchgangsloch weist vorzugsweise ebenfalls eine Sitzfläche auf, die für einen winkelstabilen Eingriff einer Knochenschraube ausgelegt ist.

Wenn die Knochenplatte drei distale Durchgangslöcher aufweist, kann der erste distale Abstand größer sein als der Abstand zwischen dem zweiten distalen Durchgangsloch und dem dritten distalen Durchgangsloch (zweiter distaler Abstand). Der zweite distale Abstand kann genauso groß sein wie der zweite proximale Abstand. Die Knochenschraube in dem ersten Durchgangsloch wird dann auf der proximalen und der distalen Seite in gleicher Weise entlastet.

Hat die Knochenplatte ein viertes proximales Durchgangsloch, so ist der Abstand zwischen dem dritten und dem vierten Durgangsloch (dritter proximaler Abstand) vorzugsweise genauso groß wie der zweite proximale Abstand. Entsprechendes kann für die distale Seite gelten, sofern dort ein viertes distales Durchgangsloch vorgesehen ist. Die erfindungsgemäße Knochenplatte kann symmetrisch sein bezogen auf eine Achse, die sich in seitlicher Richtung durch den Plattensteg erstreckt.

Die Knochenplatte kann so gestaltet sein, dass die Mittelpunkte der Durchgangslöcher mit der Längsmittellinie zusammenfallen. Die Projektion des Durchgangslochs auf die Längsmittellinie entspricht dann der tatsächlichen Position des Durchgangslochs. Bei dieser Gestaltung der Knochenplatte kann es vorkommen, dass der Knochen gespalten wird, wenn alle Knochenschrauben parallel zueinander in den Knochen eingebracht werden. Die Durchgangslöcher können deswegen winkelvariabele Sitzflächen aufweisen. An der Sitzfläche eines Durchgangslochs stützt der Kopf der Knochenschraube sich ab, wenn die Knochenschraube mit der Knochenplatte in Eingriff steht. Durch die winkelvariabele Gestaltung der Sitzflächen kann der Schraubenkopf sich unter unterschiedlichen Winkeln in dem Durchgangsloch abstützen. Beispielsweise können die Sitzflächen in dem Durchgangsloch und am Kopf der Knochenschraube eine sphärische Form haben.

Die Achsen der Durchgangslöcher rechtwinklig zur Ebene der Knochenplatte ausgerichtet sein. Das Einbringen der Knochenschrauben unter unterschiedlichen Winkeln in den Knochen kann gefördert werden, wenn die Achsen der Durchgangslöcher relativ zueinander verschränkt sind. Dabei sind die Lochachsen vorzugsweise in seitlicher Richtung geneigt.

Alternativ oder zusätzlich dazu können die Durchgangslöcher in seitlicher Richtung versetzt sein. Dies kann alle oder ausgewählte Durchgangslöcher der Knochenplatte betreffen. Beispielsweise kann das erste proximale Durchgangsloch bezogen auf die Längsmittellinie zur einen Seite hin versetzen sein und das zweite proximale Durchgangsloch zu der anderen Seite hin versetzt sein. Entsprechendes kann für das erste distale Durchgangsloch und das zweite distale Durchgangsloch gelten. Wenn die Durchgangslöcher in seitlicher Richtung versetzt zueinander sind, ist eine Projektion auf die Längsmittellinie erforderlich, um den Abstand zwischen den Durchgangslöchern ermitteln zu können.

Das Auftreten hoher Lasten am ersten proximalen Durchgangsloch und am ersten distalen Durchgangsloch ist besonders dann ausgeprägt, wenn mehrere Knochenschrauben winkelstabil mit den Durchgangslöchern verbunden sind. Es können alle Durchgangslöcher der Knochenplatte mit Sitzflächen ausgestattet sein, die für einen winkelstabilen Eingriff einer Knochenschraube ausgelegt sind.

Bei einer bevorzugten Ausführungsform ist die Verbindung zwischen dem Schraubenkopf und dem Durchgangsloch sowohl winkelstabil als auch winkelvariabel. Dies kann beispielsweise dadurch erreicht werden, dass in dem Durchgangsloch Mittel vorgesehen sind, die beim Eindrehen der Knochenschraube verformt werden, DE 43 43 117 A1. Beispielsweise kann es sich dabei um eine Lippe handeln, die von der Wand des Durchgangslochs in Richtung Zentrum des Durchgangslochs vorspringt. Am Kopf der zugehörigen Knochenschraube kann ein Gegengewinde ausgebildet sein, das zum Umformen des Materials ausgelegt ist. Um das Umformen zu erleichtern, kann die Lippe aus einem Material bestehen, das weicher ist als das Material des Schraubenkopfs. Die Lippe kann aus Reintitan bestehen, während der Kopf der Knochenschraube aus einer Titanlegierung besteht.

Eine alternative Möglichkeit zum Herstellen einer winkelvariabelen winkelstabilen Verbindung besteht darin, eines der beiden Gewinde zu segmentieren, so dass der Eingriff mit dem Gegengewinde unter unterschiedlichen Winkeln erfolgen kann, DE 43 43 117 A1, EP 1 741 397 A2. Die Sitzfläche in dem Durchgangsloch kann folglich mit einem segmentierten Gewinde versehen sein, das einen Eingriff eines Gegengewindes unter verschiedenen Winkeln ermöglicht. Das betreffende Gegengewinde kann am Kopf der zugehörigen Knochenschraube ausgebildet sein.

Die Knochenplatte kann eine gerade Form haben, so dass die Längsmittellinie sich geradlinig vom proximalen Ende bis zum distalen Ende erstreckt. Eine solche Knochenplatte ist in erster Linie für Knochen geeignet, die sich ebenfalls geradlinig erstrecken. Für anders geformte Knochen kann die Knochenplatte eine entsprechend angepasste Form haben. Die Längsmittellinie der Knochenplatte kann sich dann in einem geschwungenen Weg vom proximalen zum distalen Ende der Knochenplatte erstrecken.

Regelmäßig ist der Plattensteg, der dazu bestimmt ist, sich über die Bruchstelle des Knochens hinweg zu erstrecken, frei von Durchgangslöchern. Zur Versorgung von Splitterbrüchen, bei denen es außer dem proximalen Fragment und dem distalen Fragment noch ein weiteres Bruchstück des Knochens gibt, kann in dem Plattensteg ein Durchgangsloch vorgesehen sein, mit dem das Bruchstück relativ zu dem proximalen und dem distalen Fragment fixiert wird. Da über das Bruchstück regelmäßig nur geringe Lasten übertragen werden, kann dieses Loch einen kleineren Durchmesser haben als das erste proximale Durchgangsloch. Die proximalen und die distalen Durchgangslöcher haben vorzugsweise einen übereinstimmenden Durchmesser. Im Querschnitt sind die proximalen und die distalen Durchgangslöcher vorzugsweise kreisförmig.

Die Erstreckung des Plattenstegs entlang der Längsmittellinie ist regelmäßig mindestens so lang wie der herkömmliche Lochabstand, also der Abstand zwischen dem zweiten proximalen Durchgangsloch und dem dritten proximalen Durchgangsloch. In einer bevorzugten Ausführungsform größer als der Abstand zwischen dem zweiten proximalen Durchgangsloch und dem dritten proximalen Durchgangsloch. Weiter vorzugsweise ist die Länge des Plattenstegs größer als der Abstand zwischen dem ersten proximalen Durchgangsloch und dem zweiten proximalen Durchgangsloch. Dadurch wird erreicht, dass die erste Schraube in dem proximalen Knochenfragment einen gewissen Abstand zu der Bruchstelle des Knochens hat. Die Länge des Plattenstegs, die dem Abstand zwischen dem ersten proximalen Durchgangsloch und dem ersten distalen Durchgangsloch entspricht, kann beispielsweise zwischen 25 mm und 35 mm betragen.

Die Erfindung betrifft außerdem ein System aus einer solchen Knochenplatte und einer zugehörigen Knochenschraube. Die Knochenschraube umfasst einen Schaft und einen Schraubenkopf. Der Schraubenkopf ist mit einem Gewinde versehen, das für einen Gewindeeingriff mit der Sitzfläche des ersten proximalen Durchgangslochs ausgelegt ist. Vorzugsweise sind der Schraubenkopf und die Sitzfläche des Durchgangslochs für eine winkelvariabele und winkelstabile Verbindung ausgelegt. Das System kann mit weiteren Merkmalen fortgebildet werden, die im Zusammenhang der erfindungsgemäßen Knochenplatte beschrieben sind.

Die Erfindung wird nachfolgend unter Bezugnahme auf die beigefügten Zeichnungen anhand vorteilhafter Ausführungsformen beispielhaft beschrieben. Es zeigen:
- Fig. 1:: eine schematische Darstellung einer erfindungsgemäßen Knochenplatte, die an einem Knochen befestigt ist;
- Fig. 2:: eine erfindungsgemäße Knochenplatte in einer Ansicht von oben;
- Fig. 3:: die Ansicht aus Fig. 2 bei einer anderen Ausführungsform der Erfindung;
- Fig. 4:: die Ansicht aus Fig. 2 bei einer weiteren Ausführungsform der Erfindung;
- Fig. 5:: einen Schnitt entlang Linie A-A in Fig. 4 mit einer eingesetzten Knochenschraube; und
- Fig. 6:: die Ansicht aus Fig. 2 bei einer weiteren Ausführungsform der Erfindung.

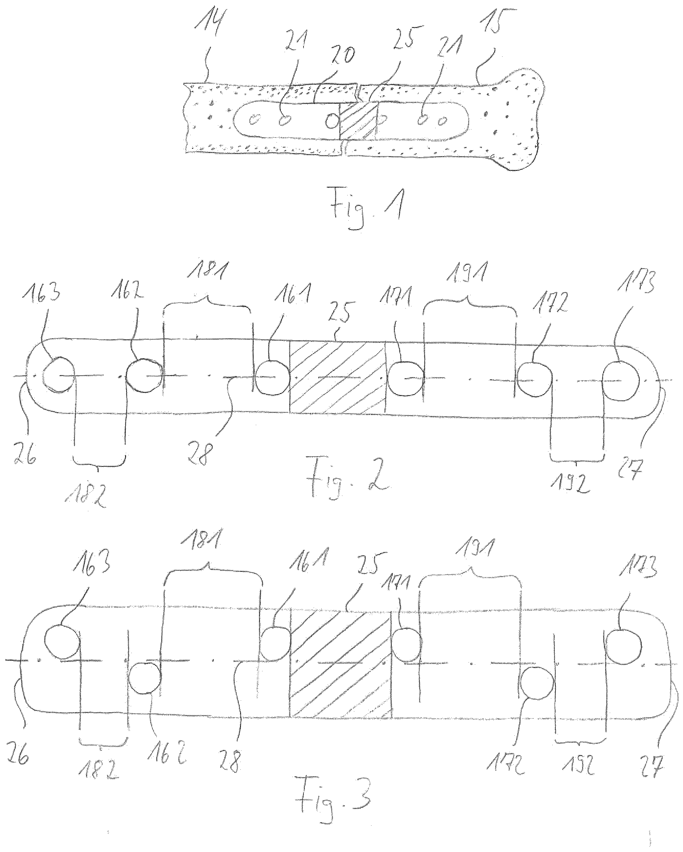

In Fig. 1 gezeigt ist ein Röhrenknochen mit einem proximalen Knochenfragment 14 und einem distalen Knochenfragment 15. Über die Bruchstelle zwischen den Knochenfragmenten 14, 15 hinweg erstreckt sich eine Knochenplatte 20, die durch insgesamt sechs Knochenschrauben 21 mit dem Knochen verbunden ist.

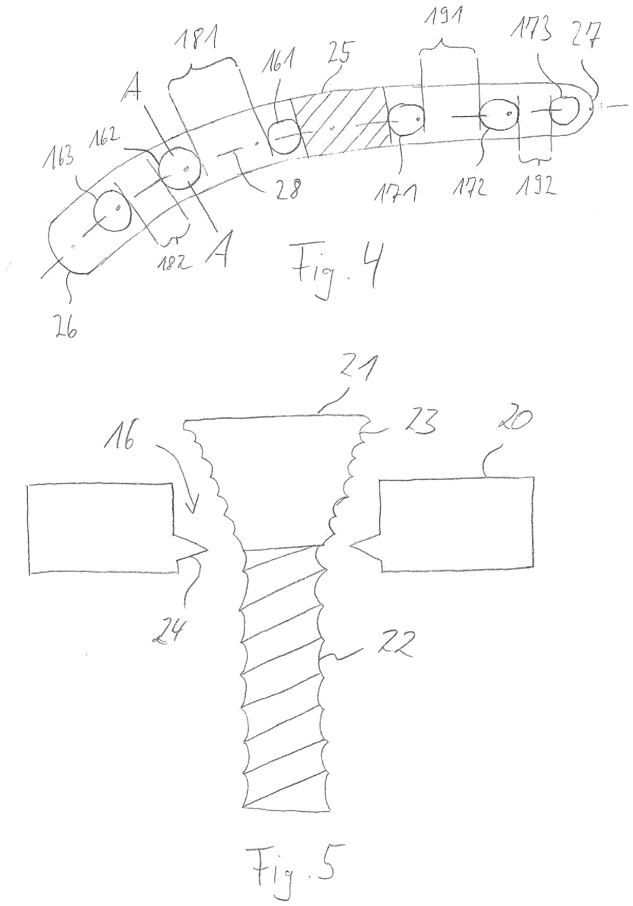

Die Verbindung wird gemäß Fig. 5 dadurch hergestellt, dass die Knochenschraube 21 durch ein Durchgangsloch 16 in der Knochenplatte 20 hindurch geführt wird. Ein Schaft 22 der Knochenschraube 21 dringt in das Material des Knochenfragments 14, 15 ein, bis der Kopf 23 der Knochenschraube 21 in das Durchgangsloch 16 der Knochenplatte 20 eintritt.

In dem Durchgangsloch ist eine Lippe 24 ausgebildet, in die ein Gewinde des Schraubenkopfs 23 eingreift, wenn die Schraube 21 hinreichend weit eingedreht wird. Die Lippe 24 besteht aus einem weicheren Material als die Knochenschraube 21, so dass die Lippe 24 sich durch den Eingriff des Schraubenkopfs verformt. Es bildet sich eine Gewindeverbindung zwischen dem Schraubenkopf 23 und der Lippe 24, deren Achse davon abhängt, unter welchem Winkel die Schraube 21 durch das Durchgangsloch 16 hindurchgeführt wurde. Die Verbindung zwischen der Schraube 21 und der Knochenplatte 20 ist also sowohl winkelvariabel als auch winkelstabil. Die Knochenschraube 21 kann aus der Titanlegierung TiAl6V4 bestehen, während das umzuformende Material der Knochenplatte 20 Reintitan ist.

Die Knochenplatte 20 ist gemäß Fig. 1 in dem proximalen Knochenfragment 14 und dem distalen Knochenfragment 15 mit jeweils drei Knochenschrauben 21 befestigt. Die Knochenschrauben 21 erstrecken sich durch drei proximale Durchgangslöcher 161, 162, 163 und drei distale Durchgangslöcher 171, 172, 173. Die Durchgangslöcher sind im Querschnitt kreisförmig. Das erste proximale Durchgangsloch 161 und das erste distale Durchgangsloch 171 schließen den Plattensteg 25 zwischen sich ein, der sich über die Bruchstelle hinweg erstreckt, wenn die Knochenplatte 20 mit dem Knochen verbunden ist. Die Länge des Plattenstegs 25 entspricht dem Abstand der einander zugewandten Enden des ersten proximalen Durchgangsloch 161 und des ersten distalen Durchgangsloch 171.

Das zweite proximale Durchgangsloch 162 und das dritte proximale Durchgangsloch 163 sowie das zweite distale Durchgangsloch 172 und das dritte distale Durchgangsloch 173 sind in einer jeweils größeren Entfernung zu dem Plattensteg 25 angeordnet. Das dritte proximale Durchgangsloch 163 ist nahe dem proximalen Ende 26 der Knochenplatte 20 angeordnet. Das dritte distale Durchgangsloch 173 ist nahe dem distalen Ende 27 der Knochenplatte 20 angeordnet.

Die Knochenplatte 20 erstreckt sich geradlinig vom proximalen Ende 26 bis zum distalen Ende 27. Die Längsmittellinie 28, die sich vom proximalen Ende 26 bis zum distalen Ende 27 mittig durch die Knochenplatte 20 hindurch erstreckt, ist folglich ebenfalls geradlinig. Die Durchgangslöcher 161, 162, 163, 171, 172, 173 sind mittig in der Knochenplatte 20 angeordnet, was bedeutet, dass der Mittelpunkt jedes Durchgangslochs mit der Längsmittellinie 28 zusammenfällt.

Der Abstand zwischen dem ersten proximalen Durchgangsloch 161 und dem zweiten proximalen Durchgangsloch 162 wird als erster proximaler Abstand 181 bezeichnet. Der Abstand zwischen dem zweiten proximalen Durchgangsloch 162 und dem dritten proximalen Durchgangsloch 163 wird als zweiter proximaler Abstand 182 bezeichnet. Der erste proximale Abstand 181 ist um etwa den Faktor 1,6 größer als der zweite proximale Abstand 182. Entsprechendes gilt auf der distalen Seite der Knochenplatte 20. Der Abstand zwischen dem ersten distalen Durchgangsloch 171 und dem zweiten distalen Durchgangsloch 172 wird als erster distaler Abstand 191 bezeichnet. Der Abstand zwischen dem zweiten distalen Durchgangsloch 172 und dem dritten distalen Durchgangsloch 173 wird als zweiter distalen Abstand 192 bezeichnet. Der erste distale Abstand 191 ist um etwa den Faktor 1,6 größer als der zweite distale Abstand 192. Die Knochenplatte ist symmetrisch sowohl bezogen auf die Längsmittellinie 28 als auch bezogen auf die in Querrichtung dazu liegende Achse, die sich in seitlicher Richtung durch den Plattensteg 25 erstreckt.

Bei der in Fig. 3 gezeigten Ausführungsform sind die Durchgangslöcher 161, 162, 163, 171, 172, 173 in seitlicher Richtung versetzt. Die Mittelpunkte der Durchgangslöcher fallen also nicht mit der Längsmittellinie 28 zusammen, sondern sind zwischen der Längsmittellinie 28 und dem seitlicher Rand der Knochenplatte 20 angeordnet. Diese Anordnung der Durchgangslöcher hat den Vorteil, dass die Knochenschrauben 21 nicht entlang einer Achse des Knochens in den Knochen eingeschraubt werden können. Das Risiko, den Knochen zu spalten, wird dadurch vermindert.

Für die Bestimmung des Abstands zwischen den Durchgangslöchern werden die Durchgangslöcher in eine gedachte Position projiziert, in der der Mittelpunkt des Durchgangslochs auf der Längsmittellinie 28 liegt. Bei dieser Ausführungsform gilt ebenfalls, dass der erste proximale Abstand 181 um etwa den Faktor 1,6 größer ist als der zweite proximale Abstand 182 und dass der erste distale Abstand 191 um etwa den Faktor 1,6 größer ist als der zweite distale Abstand 192.

In Fig. 4 hat die Knochenplatte 20 eine gebogene Form, so dass die Längsmittellinie 28 sich ebenfalls in einem Bogen erstreckt. Die Durchgangslöcher 161, 162, 163, 171, 172, 173 sind mittig angeordnet, so dass die Mittelpunkte jeweils auf der Längsmittellinie 28 liegen. Der Abstand zweier Durchgangslöcher ergibt sich aus der entlang der Längsmittellinie 28 gemessenen Strecke zwischen den zwei einander zugewandten Enden der beiden Durchgangslöcher. Auch bei dieser Ausführungsform ist der erste proximale Abstand 181 um etwa den Faktor 1,6 größer als der zweite proximale Abstand 182. Der erste distale Abstand 191 ist um etwa den Faktor 1,6 größer als der zweite distale Abstand 192.

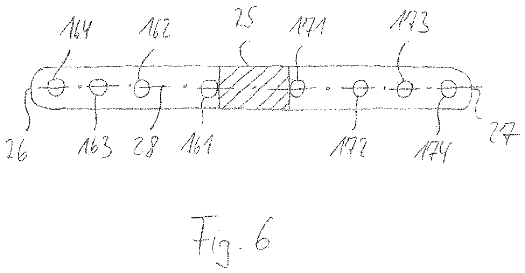

Fig. 6 zeigt eine Knochenplatte 20, die sich geradlinig vom proximalen Ende 26 bis zum distalen Ende 27 erstreckt und bei der die Mittelpunkte aller Durchgangslöcher mit der Längsmittellinie 28 zusammenfallen. Die Knochenplatte 20 hat zusätzlich ein viertes proximales Durchgangsloch 164 und ein viertes distales Durchgangsloch 174. Der dritte proximale Abstand zwischen den Durchgangslöchern 163 und 164 ist genauso groß wie der zweite proximale Abstand zwischen den Durchgangslöchern 162 und 163. Der erste proximale Abstand zwischen den Durchgangslöchern 161 und 162 ist um etwa den Faktor 1,6 größer. Entsprechendes gilt für die Abstände zwischen den distalen Durchgangslöchern. Die Länge des Plattenstegs 25 ist etwas größer als der erste proximale Abstand. Die Knochenplatte ist symmetrisch sowohl bezogen auf die Längsmittellinie 28 als auch bezogen auf die in Querrichtung dazu liegende Achse, die sich in seitlicher Richtung durch den Plattensteg 25 erstreckt.

Indem der erste proximale Abstand 181 größer ist als der zweite proximale Abstand 182 hat das erste Durchgangsloch 161 neben dem Plattensteg eine vergleichsweise isolierte Position. Es hat sich gezeigt, dass die über das erste proximale Durchgangsloch 161 und eine dort eingeführte Knochenschraube 21 übertragenen Lasten durch die erfindungsgemäße Anordnung der Durchgangslöcher vermindert werden. Das Gleiche gilt für das erste distale Durchgangsloch 171, das auf der anderen Seite des Plattenstegs 25 angeordnet ist.

## Patentansprüche

1. Knochenplatte mit einer Mehrzahl von Durchgangslöchern (161, 162, 163, 171, 172, 173), die sich von einer Oberseite bis zu einer knochenseitigen Unterseite der Knochenplatte (20) erstrecken, mit einem über eine Bruchstelle des Knochens hinweg anzuordnenden Plattensteg (25), der sich von einem ersten proximalen Durchgangsloch (161) bis zu einem ersten distalen Durchgangsloch (171) erstreckt, wobei ein zweites proximales Durchgangsloch (162) einen größeren Abstand zu dem Plattensteg (25) hat als das erste proximale Durchgangsloch (161) und wobei ein drittes proximales Durchgangsloch (163) einen größeren Abstand zu dem Plattensteg (25) hat als das zweite proximale Durchgangsloch (162), wobei das erste proximale Durchgangsloch (161) eine Sitzfläche (24) aufweist, die für einen winkelstabilen Eingriff einer Knochenschraube (21) ausgelegt ist, **dadurch gekennzeichnet, dass** in einer Projektion auf eine Längsmittellinie (28) der Knochenplatte (20) der erste proximale Abstand (181) zwischen dem ersten proximalen Durchgangsloch (161) und dem zweiten proximalen Durchgangsloch (162) größer ist als der zweite proximale Abstand (182) zwischen dem zweiten proximalen Durchgangsloch (162) und dem dritten proximalen Durchgangsloch (163).

2. Knochenplatte nach Anspruch 1, **dadurch gekennzeichnet, dass** der erste proximale Abstand (181) um einen Faktor zwischen 1,2 und 2, vorzugsweise um einen Faktor zwischen 1,4 und 1,8 größer ist als der zweite proximale Abstand (182).

3. Knochenplatte nach Anspruch 1 oder 2, **dadurch gekennzeichnet, dass** sie zusätzlich zum ersten distalen Durchgangsloch (171) ein zweites distales Durchgangsloch (172) aufweist.

4. Knochenplatte nach Anspruch 3, **dadurch gekennzeichnet, dass** der erste distale Abstand (191) zwischen dem ersten distalen Durchgangsloch (171) und dem zweiten distalen Durchgangsloch (172) genauso groß ist wie der erste proximale Abstand (181).

5. Knochenplatte nach Anspruch 3 oder 4, **dadurch gekennzeichnet, dass** sie ein drittes distales Durchgangsloch (173) aufweist, wobei der erste distale Abstand (191) größer ist als der zweite distale Abstand (192) zwischen dem zweiten distalen Durchgangsloch (172) und dem dritten distalen Durchgangsloch (173).

6. Knochenplatte nach einem der Ansprüche 1 bis 5, **dadurch gekennzeichnet, dass** sie ein viertes proximales Durchgangsloch (164) aufweist und dass der Abstand zwischen dem dritten proximalen Durchgangsloch (163) und dem vierten proximalen Durchgangsloch (164) genauso groß ist wie der zweite proximale Abstand (182).

7. Knochenplatte nach einem der Ansprüche 1 bis 6, **dadurch gekennzeichnet, dass** die Durchgangslöcher (161, 162, 163, 171, 172, 173) winkelvariabele Sitzflächen (24) aufweisen.

8. Knochenplatte nach einem der Ansprüche 1 bis 7, **dadurch gekennzeichnet, dass** das erste proximale Durchgangsloch (161) und das zweite proximale Durchgangsloch (162) bezogen auf die Längsmittellinie (28) in seitlicher Richtung versetzt angeordnet sind.

9. Knochenplatte nach einem der Ansprüche 1 bis 8, **dadurch gekennzeichnet, dass** alle Durchgangslöcher (161, 162, 163, 171, 172, 173) Sitzflächen (24) aufweisen, die für einen Gewindeeingriff mit einem am Kopf (23) einer Knochenschraube (21) ausgebildeten Gewinde ausgelegt sind.

10. Knochenplatte nach einem der Ansprüche 1 bis 9, **dadurch gekennzeichnet, dass** der Plattensteg (25) eine Länge hat, die größer ist als der zweite proximale Abstand (182).

11. System aus eine Knochenplatte nach einem der Ansprüche 1 bis 10 und einer Knochenschraube, **dadurch gekennzeichnet, dass** der Kopf (23) der Knochenschraube (21) mit einem Gewinde versehen ist, das für einen Gewindeeingriff mit der Sitzfläche (24) des ersten proximalen Durchgangslochs (161) ausgelegt ist.

12. System nach Anspruch 11, **dadurch gekennzeichnet, dass** der Kopf (23) der Knochenschraube (21) und die Sitzfläche (24) des Durchgangslochs (161, 162, 163, 171, 172, 173) für eine winkelvariable und winkelstabile Verbindung ausgelegt sind.

## Claims

1. Bone plate with a plurality of through-holes (161, 162, 163, 171, 172, 173) which extend from an upper side of the bone plate (20) to a lower side thereof facing the bone, with a plate bridge (25) which is to be arranged across a fracture site of the bone and which extends from a first proximal through-hole (161) to a first distal through-hole (171), wherein a second proximal through-hole (162) is at a greater distance from the plate bridge (25) than the first proximal through-hole (161), and wherein a third proximal through-hole (163) is at a greater distance from the plate bridge (25) than the second proximal through-hole (162), wherein the first proximal through-hole (161) has a seat surface (24) which is designed for engagement of a bone screw (21) at a stable angle, **characterized in that**, in a projection onto a longitudinal centre line (28) of the bone plate (20), the first proximal distance (181) between the first proximal through-hole (161) and the second proximal through-hole (162) is greater than the second proximal distance (182) between the second proximal through-hole (162) and the third proximal through-hole (163).

2. Bone plate according to Claim 1, **characterized in that** the first proximal distance (181) is greater than the second proximal distance (182) by a factor of between 1.2 and 2, preferably by a factor of between 1.4 and 1.8.

3. Bone plate according to Claim 1 or 2, **characterized in that**, in addition to the first distal through-hole (171), it has a second distal through-hole (172) .

4. Bone plate according to Claim 3, **characterized in that** the first distal distance (191) between the first distal through-hole (171) and the second distal through-hole (172) is exactly the same size as the first proximal distance (181).

5. Bone plate according to Claim 3 or 4, **characterized in that** it has a third distal through-hole (173), wherein the first distal distance (191) is greater than the second distal distance (192) between the second distal through-hole (172) and the third distal through-hole (173).

6. Bone plate according to one of Claims 1 to 5, **characterized in that** it has a fourth proximal through-hole (164), and **in that** the distance between the third proximal through-hole (163) and the fourth proximal through-hole (164) is exactly the same size as the second proximal distance (182).

7. Bone plate according to one of Claims 1 to 6, **characterized in that** the through-holes (161, 162, 163, 171, 172, 173) have seat surfaces (24) of variable angles.

8. Bone plate according to one of Claims 1 to 7, **characterized in that** the first proximal through-hole (161) and the second proximal through-hole (162) are arranged so as to be offset in the lateral direction with respect to the longitudinal centre line (28).

9. Bone plate according to one of Claims 1 to 8, **characterized in that** all of the through-holes (161, 162, 163, 171, 172, 173) have seat surfaces (24) which are designed for threaded engagement with a thread formed on the head (23) of a bone screw (21).

10. Bone plate according to one of Claims 1 to 9, **characterized in that** the plate bridge (25) has a length that is greater than the second proximal distance (182).

11. System composed of a bone plate according to one of Claims 1 to 10 and of a bone screw, **characterized in that** the head (23) of the bone screw (21) is provided with a thread which is designed for threaded engagement with the seat surface (24) of the first proximal through-hole (161).

12. System according to Claim 11, **characterized in that** the head (23) of the bone screw (21) and the seat surface (24) of the through-hole (161, 162, 163, 171, 172, 173) are designed for an angle-variable and angle-stable connection.

## Revendications

1. Plaque d'ostéosynthèse comprenant une pluralité de trous de passage (161, 162, 163, 171, 172, 173) qui s'étendent depuis un côté supérieur jusqu'à un côté inférieur côté os de la plaque d'ostéosynthèse (20), une languette de plaque (25) devant être disposée par-dessus et au-delà d'une zone de fracture de l'os, qui s'étend depuis un premier trou de passage proximal (161) jusqu'à un premier trou de passage distal (171), un deuxième trou de passage proximal (162) étant plus éloigné de la languette de plaque (25) que le premier trou de passage proximal (161) et un troisième trou de passage proximal (163) étant plus éloigné de la languette de plaque (25) que le deuxième trou de passage proximal (162), le premier trou de passage proximal (161) présentant une surface d'assise (24) qui est conçue pour permettre l'engagement angulaire stable d'une vis à os (21), **caractérisée en ce que**, en projection sur un axe médian longitudinal (28) de la plaque d'ostéosynthèse (20), la première distance proximale (181) entre le premier trou de passage proximal (161) et le deuxième trou de passage proximal (162) est supérieure à la deuxième distance proximale (182) entre le deuxième trou de passage proximal (162) et le troisième trou de passage proximal (163).

2. Plaque d'ostéosynthèse selon la revendication 1, **caractérisée en ce que** la première distance proximale (181) est supérieure à la deuxième distance proximale (182) d'un facteur compris entre 1,2 et 2, de préférence d'un facteur compris entre 1,4 et 1,8.

3. Plaque d'ostéosynthèse selon la revendication 1 ou 2, **caractérisée en ce qu'**elle présente en outre un deuxième trou de passage distal (172) en plus du premier trou de passage distal (171).

4. Plaque d'ostéosynthèse selon la revendication 3, **caractérisée en ce que** la première distance distale (191) entre le premier trou de passage distal (171) et le deuxième trou de passage distal (172) est aussi grande que la première distance proximale (181) .

5. Plaque d'ostéosynthèse selon la revendication 3 ou 4, **caractérisée en ce qu'**elle présente un troisième trou de passage distal (173), la première distance distale (191) étant supérieure à la deuxième distance distale (192) entre le deuxième trou de passage distal (172) et le troisième trou de passage distal (173).

6. Plaque d'ostéosynthèse selon l'une quelconque des revendications 1 à 5, **caractérisée en ce qu'**elle présente un quatrième trou de passage proximal (164) et **en ce que** la distance entre le troisième trou de passage proximal (163) et le quatrième trou de passage proximal (164) est aussi grande que la deuxième distance proximale (182).

7. Plaque d'ostéosynthèse selon l'une quelconque des revendications 1 à 6, **caractérisée en ce que** les trous de passage (161, 162, 163, 171, 172, 173) présentent des surfaces d'assise (24) d'angles variables.

8. Plaque d'ostéosynthèse selon l'une quelconque des revendications 1 à 7, **caractérisée en ce que** le premier trou de passage proximal (161) et le deuxième trou de passage proximal (162) sont disposés de manière décalée dans la direction latérale par rapport à l'axe médian longitudinal (28) .

9. Plaque d'ostéosynthèse selon l'une quelconque des revendications 1 à 8, **caractérisée en ce que** tous les trous de passage (161, 162, 163, 171, 172, 173) présentent des surfaces d'assise (24) qui sont conçues pour un engagement par filetage avec un filetage réalisé sur la tête (23) d'une vis à os (21) .

10. Plaque d'ostéosynthèse selon l'une quelconque des revendications 1 à 9, **caractérisée en ce que** la languette de plaque (25) présente une longueur qui est supérieure à la deuxième distance proximale (182) .

11. Système constitué d'une plaque d'ostéosynthèse selon l'une quelconque des revendications 1 à 10 et d'une vis à os, **caractérisé en ce que** la tête (23) de la vis à os (21) est pourvue d'un filetage qui est conçu pour s'engager par filetage avec la surface d'assise (24) du premier trou de passage proximal (161).

12. Système selon la revendication 11, **caractérisé en ce que** la tête (23) de la vis à os (21) et la surface d'assise (24) du trou de passage (161, 162, 163, 171, 172, 173) sont conçues pour une liaison à angles variables et à angles stables.
